# EUROPÄISCHE PATENTANMELDUNG

(11) **EP 1 364 993 A1**
(43) Veröffentlichungstag der Anmeldung: **26.11.2003**
(21) Anmeldenummer: 03010362.6
(22) Anmeldetag: 08.05.2003
(51) Int. Cl.: C09B 1/28, C09B 1/20, C09B 1/32, C07C 221/00, C07C 225/34

(54) **Verfahren zur Herstellung von N,N'-disubsituierten 1,4-Diaminoanthrachinonen**

(30) Priorität: 21.05.2002 DE 10222819
(71) Anmelder: Bayer Aktiengesellschaft, 51368 Leverkusen (DE)
(72) Erfinder: Feldhues, Ulrich, Dr., 51465 Bergisch Gladbach (DE); Stawitz, Josef-Walter, Dr., 51519 Odenthal (DE); Naseband, Julius, 51519 Odenthal (DE)

(57) **Zusammenfassung**

Verfahren zur Herstellung von N,N'-disubstiuierten 1,4-Diaminoanthrachinonen 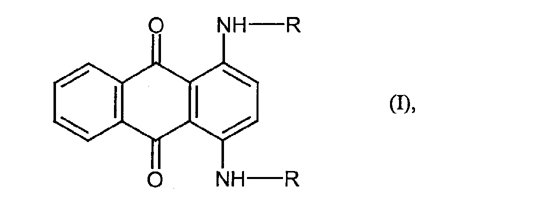 worin
- R: ein aliphatischer oder aromatischer Rest ist,
dadurch gekennzeichnet, dass man 1,4-Dihydroxyanthrachinone mit aliphatischen oder aromatischen Aminen in Gegenwart eines gut-wasserlöslichen dipolar-aprotischen Lösungsmittels (z.B. NMP) und eines zweiten davon verschiedenen Lösungsmittels umsetzt.

## Beschreibung

Die Erfindung betrifft ein Verfahren zur Herstellung von N,N'-disubstituierten 1,4-Diaminoanthrachinonen sowie die Verwendung so hergestellter Verbindungen zum Massefärben von Kunststoffen.

N,N'-disubstituierte 1,4-Diaminoanthrachinone sind beispielsweise als Farbstoffe für Kunststoffe und Synthesefasern als auch für Vorprodukte zur Herstellung von Wollfarbstoffen bekannt. Bislang wurden 1,4-Diaminoanthrachinone hergestellt, indem man 1,4-Dihydroxyanthrachinon (Chinizarin) gegebenenfalls im Gemisch mit 2,3-Dihydro-1,4-dihydroxyanthrachinon (Leukochinizarin) mit Aminen umsetzt, wobei die Reaktion gegebenenfalls in Gegenwart von Kondensationshilfsmitteln durchgeführt wurde. Als Kondensationshilfsmittel sind beispielsweise Salzsäure (DE-A-2 342 469), Essigsäure (US-A-4 083 683) oder Hydroxycarbonsäuren (DE-A 195 17 071) bekannt. Doch selbst bei Verwendung dieser Hilfsmittel sind sowohl die Reaktionszeiten als auch die Ausbeuten nicht optimal. Nach dem Stand der Technik entstehen zum Teil im Reaktionsmedium unlösliche Nebenprodukte, die sich dann gegebenenfalls als unlösliche Verunreinigung im Hauptprodukt wiederfinden. Ein weiterer Nachteil sind erhebliche Viskositätsprobleme im Reaktionsverlauf, die zu schlechten Raum-/Zeit-Ausbeuten führen.

DE-A 199 36 282 beschreibt die Herstellung von Amino-hydroxy-anthrachinonen aus Chlor-hydroxyanthrachinonen u.a. in Gegenwart gut-wasserlöslicher dipolar-aprotischer Lösungsmittel. Die Hydroxygruppe wird unter diesen Bedingungen nicht gegen eine Aminogruppe ausgetauscht.

Aufgabe der vorliegenden Erfindung ist die Bereitstellung eines Verfahrens, welches die Nachteile des Standes der Technik nicht mehr besitzt.

Es wurde nun ein Verfahren zur Herstellung von N,N'-disubstituierten 1,4-Diaminoanthrachinonen gefunden, das dadurch gekennzeichnet ist, dass man 1,4-Dihydroxyanthrachinon (Chinizarin) mit aliphatischen oder aromatischen Aminen in Gegenwart eines dipolar aprotischen Lösungsmittels mit einer Wasserlöslichkeit von wenigstens 30 Gew.-% bei 20°C, bezogen auf die wässrige Lösung, und wenigstens eines zweiten davon verschiedenen Lösungsmittels umsetzt.

Das dipolar-aprotische Lösungsmittel ist vorzugsweise mit Wasser mischbar. Bevorzugt sind gegebenenfalls substituiertes Formamid oder Sulfoxid wie Dimethylformamid, Dimethylsulfoxid, ein gegebenenfalls substituiertes Lactam oder Lacton, besonders bevorzugt Butyrolacton, N-Methyl-2-pyrrolidon oder Caprolactam, ganz besonders bevorzugt N-Methyl-2-pyrrolidon.

Das zweite Lösungsmittel besitzt vorzugsweise eine Löslichkeit in Wasser von kleiner 20 Gew.-% bei 20°C, bezogen auf die wässrige Lösung, besonders bevorzugt eine Löslichkeit in Wasser von 5 Gew.-% bis 15 Gew.-% bei 20°C. Das zweite Lösungsmittel bildet vorzugsweise mit Wasser ein Azeotrop. Das zweite Lösungsmittel ist vorzugsweise mit dem dipolar-aprotischen Lösungsmittel mischbar. Geeignete zweite Lösungsmittel sind beispielsweise aliphatische Alkohole, wie 1-Butanol, 2-Methyl-1-propanol, 2-Butanol, sowie ggf. substituierte Aromaten wie Dichlorbenzol, Toluol, Xylol u.a. Auch im Überschuss eingesetztes Amin kann als zweites Lösungsmittel verwendet werden. Besonders bevorzugt sind protische Lösungsmittel, ganz besonders bevorzugt aliphatische Alkohole, insbesondere 1-Butanol.

Vorzugsweise wird das dipolar-aprotische Lösungsmittel und das zweite Lösungsmittel im Verhältnis 2 : 98 bis 98 : 2, insbesondere von 5 : 95 bis 50 : 50, ganz besonders bevorzugt von 10 : 90 bis 20 : 80 eingesetzt.

Das im erfindungsgemäßen Verfahren eingesetzte 1,4-Dihydroxyanthrachinon (Chinizarin) wird bevorzugt im Gemisch mit seiner Leukoform, dem 2,3-Dihydro-1,4-dihydroxyanthrachinon (Leukochinizarin), vorzugsweise im Verhältnis 95 : 5 bis 25 : 75, insbesondere von 90: 10 bis 50 : 50, ganz besonders bevorzugt von 90 : 10 bis 70 : 30 eingesetzt.

Das Gemisch aus Leukochinizarin und Chinizarin kann beispielsweise aus dem Chinizarin durch Zugabe von Reduktionsmitteln wie Zinkstaub oder Natriumdithionit in situ gebildet werden. Die Anthrachinonverbindungen Chinizarin und ihre Leukoform können aber auch separat hergestellt werden.

In einer bevorzugten Variante wird die Leukoform erst im Reaktionsverlauf erzeugt bzw. zugegeben. Besonders bevorzugt ist es, die Leukoform gelöst in dem dipolar-aprotischen Lösungsmittel zuzugeben. Besonders vorteilhaft ist es, die Leukoform der bereits erwärmten, vorzugsweise größer 50°C warmen Reaktionsmischung zuzugeben.

Die bevorzugten, bei dem erfindungsgemäßen Verfahren zum Einsatz kommenden aliphatischen oder aromatischen Amine, sind primär. Die aliphatischen Amine können beispielsweise gesättigt, ungesättigt, verzweigt oder geradkettig sein. Besonders bevorzugte aliphatische Amine sind beispielsweise solche der nachstehenden Formeln:

H₂N - CH₃ , H₂N-CH₂-CH₃ , H₂N-CH₂-CH₂-CH₃ ,

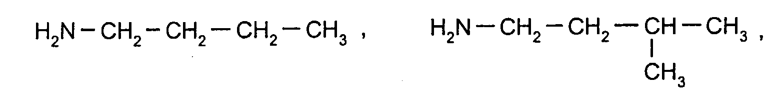

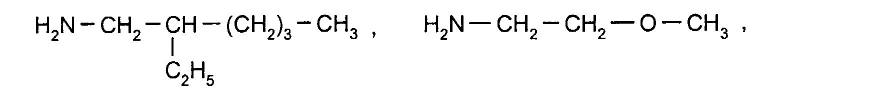

H₂N-CH₂-CH₂-CH₂-O-CH₃, H₂N-(CH₂)₃- O - C₂H₅ ,

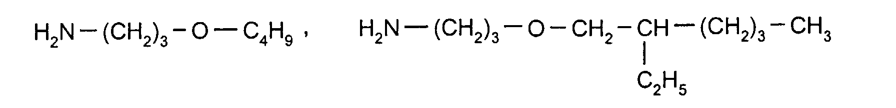

und

H₂N ―(CH₂)₃ ― O ― C₁₈H₃₇ .

Besonders vorteilhaft wird das erfindungsgemäße Verfahren jedoch zur Herstellung von N,N'-disubstituierten 1,4-Diarylaminoanthrachinonen verwendet, bei denen die aromatischen Amine primär sind, und insbesondere der Formel 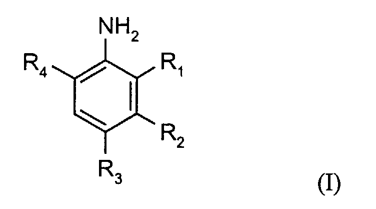 entsprechen, worin
- R₁, R₃ und R₄: unabhängig voneinander H oder C₁-C₁₂-Alkyl, insbesondere C₁-C₄-Alkyl, bedeuten und
- R₂: für H oder -SO₂-NH-R₅ steht, wobei R₅ für gegebenenfalls substituiertes Aryl, insbesondere C₆-C₁₀-Aryl, wie Phenyl oder Naphthyl, oder Alkyl, insbesondere C₁-C₄-Alkyl, wie Methyl, Ethyl, Propyl oder Butyl, steht und als mögliche Substituenten C₁-C₄-Alkyl, OH, Halogen, C₁-C₄-Alkoxy auch C₆-C₁₀-Aryloxy bevorzugt sind.

Besonders bevorzugt sind aromatische Amine der Formel (I), worin R₁ bis R₄ die in der nachstehenden Tabelle genannten Bedeutungen haben.

**Tabelle**

| **R**_{**1**} | **R**_{**2**} | **R**_{**3**} | **R**_{**4**} |
|---|---|---|---|
| H | H | CH₃ | H |
| H | H | t.-Bu | H |
| H | H | H | CH₃ |
| CH₃ | H | H | C₂H₅ |
| CH₃ | H | H | CH₃ |
| C₂H₅ | H | H | C₂H₅ |
| CH₃ | H | CH₃ | CH₃ |
| C₂H₅ | H | CH₃ | C₂H₅ |
| CH₃ | SO₂NH-C₆H₅ | CH₃ | CH₃ |

In einer besonders bevorzugten Ausführungsform wird das erfindungsgemäße Verfahren in Gegenwart von Borsäure durchgeführt. Vorzugsweise wird diese in einer Menge von 0,1 bis 1 Moläquivalent, bezogen auf die auszutauschende Hydroxygruppe des Anthrachinons (Gesamtmenge an Chinizarin und Leukochinizarin), insbesondere 0,25 bis 0,8 Moläquivalente, eingesetzt.

Selbstverständlich kann man das erfindungsgemäße Verfahren auch in Gegenwart zusätzlicher Kondensationsmittel durchführen, wie sie beispielsweise in der DE-A-2 342 469 (Salzsäure), US-A-4 083 683 (Essigsäure) oder DE-A 195 17 071 (Hydroxycarbonsäuren) beschrieben werden. Vorzugsweise wird das erfindungsgemäße Verfahren ohne entsprechende Zusätze durchgeführt.

Das erfindungsgemäße Verfahren wird vorzugsweise bei einer Temperatur von 60 bis 200°C, vorzugsweise 90 bis 160°C, insbesondere 110 bis 140°C durchgeführt.

Wasser wird vorzugsweise während der Reaktion entfernt, beispielsweise durch Destillation an einem Wasserabscheider. Die Destillation kann unter Normaldruck, unter vermindertem Druck oder auch gegen erhöhten Druck durchgeführt werden.

Das erfindungsgemäße Verfahren läuft deutlich selektiver ab als Verfahren nach dem Stand der Technik. Besonders groß ist der Gewinn an Ausbeute bei Verwendung aromatischer Amine der Formel (I). Nach Beendigung der erfindungsgemäßen Kondensationsreaktion besitzt die Reaktionsmischung in der Regel eine Temperatur, die vorzugsweise oberhalb der Siedetemperatur von Wasser liegt.

Auch Temperaturen darunter sind vorteilhaft, sofern der äußere Druck vermindert worden ist. Die Reaktionsmischung wird nach beendeter Reaktion vorzugsweise abgekühlt.

Zur Oxidation von gegebenenfalls vorhandenen Leukoverbindungen kann vorzugsweise Luft durch die Reaktionsmischung geleitet werden. Die Oxidation kann jedoch auch mit anderen Oxidationsmittel als Sauerstoff durchgeführt werden. Eine Oxidation kann ggf. auch entfallen. Danach folgt in der Regel die Isolierung der 1,4-Diaminoanthrachinonverbindung, die im allgemeinen mit aliphatischen Alkoholen wie Methanol, Ethanol, Propanol, Butanol oder mit Wasser oder Alkoholgemischen gefällt wird. Ein Vorteil des erfindungsgemäßen Verfahrens ist der, dass die gewünschten Produkte auch ohne Fällung mit einem aliphatischen Alkohol und/oder Wasser in ausgezeichneten Ausbeuten und Qualitäten erhalten werden.

Die 1,4-Diaminoanthrachinonverbindung wird vorzugsweise filtriert und vorzugsweise mit den genannten Alkoholen gewaschen. In der Regel schließen sich daran noch Wasserwäschen und schließlich die Trocknung an. Auch die Fällung mit wässriger Salzsäure wie sie aus US-A 4.083.683, Beispiel 1, bekannt ist, kann zur Isolierung verwendet werden.

Die in den Beispielen angegebenen Ausbeuten sind auf die eingesetzte Gesamtmenge an Chinizarin und Leukochinizarin bezogen.

Das erfindungsgemäße Verfahren zeichnet sich durch eine verbesserte Verfahrensführung (z.B. niedrige Viskosität), durch eine exzellente Raum-Zeit-Ausbeute und durch verbesserte Verfahrensprodukte aus.

Die nach dem erfindungsgemäßen Verfahren hergestellten Farbstoffe eignen sich besonders zum Massefärben von Kunststoffen.

Unter Massefärben werden hierbei insbesondere Verfahren verstanden, bei denen der Farbstoff in die geschmolzene Kunststoffinasse eingearbeitet wird, z.B. unter Zuhilfenahme eines Extruders, oder bei dem der Farbstoff bereits Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Polyester, Polyamide sowie Polyolefine, insbesondere Polyethylen und Polypropylen oder Polycarbonate.

Geeignete Vinylpolymere sind Polystyrol, Styrol-Acrylnitril-Copolymere, Styrol-Butadien-Copolymere, Styrol-Butadien-Acrylnitril-Terpolymere, Polymethymethacrylat, Polyvinylchlorid u.a.

Weiterhin geeignet sind u.a. Polyester wie beispielsweise Polyethylenterephthalate, Polycarbonate und Celluloseester.

Bevorzugt sind Polystyrol, Styrol-Mischpolymere, Polycarbonate, Polymethacrylate und Polyamide. Besonders bevorzugt ist Polystyrol, Polyethylen und Polypropylen.

Die erwähnten hochmolekularen Verbindungen können einzeln oder in Gemischen, als plastische Massen oder Schmelzen vorliegen.

Die nach dem erfindungsgemäßen Verfahren erhaltenen Farbstoffe werden vorzugsweise in feinverteilter Form zur Anwendung gebracht, wobei Dispergiermittel mitverwendet werden können aber nicht müssen.

Wird der nach dem erfindungsgemäßen Verfahren erhaltene Farbstoff nach der Polymerisation eingesetzt, so wird er vorzugsweise mit dem Kunststoffgranulat trocken vermischt oder vermahlen und dieses Gemisch z.B. auf Mischwalzen oder in Schnecken plastifiziert und homogenisiert. Man kann die erhaltenen Farbstoffe aber auch der schmelzflüssigen Masse zugeben und diese durch Rühren homogen verteilen. Das derart vorgefärbte Material wird dann wie üblich z.B. durch Verspinnen zu Borsten, Fäden usw. oder durch Extrusion oder im Spritzguss-Verfahren zu Formteilen weiterverarbeitet.

Da die Farbstoffe gegenüber Polymerisationskatalysatoren insbesondere Peroxiden, beständig sind, ist es auch möglich, den Farbstoff den monomeren Ausgangsmaterialien für die Kunststoffe zuzusetzen und dann in Gegenwart von Polymerisationskatalysatoren zu polymerisieren. Dazu wird der Farbstoff vorzugsweise in den monomeren Komponenten gelöst oder mit ihnen innig vermischt.

Die nach dem erfindungsgemäßen Verfahren erhaltenen Farbstoffe werden vorzugsweise zum Färben der genannten Polymeren in Mengen von 0,0001 bis 1 Gew.-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Nach dem erfindungsgemäßen Färbe-Verfahren erhält man transparente bzw. gedeckte brillante blaue bis grüne Färbungen mit guter Hitzebeständigkeit sowie guter Licht-, Wetter- und Sublimierechtheit.

In das erfindungsgemäße Färbe-Verfahren können auch Mischungen der Farbstoffe der Formel (I) mit anderen Farbstoffen und/oder anorganischen bzw. organischen Pigmenten eingesetzt werden.

Die Erfindung wird erläutert, jedoch nicht beschränkt durch die folgenden Beispiele, in denen die Teile gewichtsmäßig angegeben sind, Prozentangaben Gewichtsprozente (Gew.-%) bedeuten.

### Beispiele

### Beispiel 1

In einen 500 ml 4-Halskolben werden 165 ml n-Butanol eindosiert. Unter Rühren werden 50,5 g p-Toluidin, 15,5 g Borsäure und 42 g 99,5%iges Chinizarin eingetragen. Der Ansatz wird am Wasserabscheider zum Rückfluss erhitzt. Nach 10 min Rückfluss werden 7,5 g 93,5%iges Dihydrochinizarin gelöst in 22,5 ml N-Methyl-2-pyrrolidon innerhalb 3h zugetropft. Nach weiteren 9h am Rückfluss wird der Ansatz abgekühlt, filtriert, mit 80 ml 60°C heißem n-Butanol, danach portionsweise mit 200ml 60°C heißem Methanol, danach mit 2000ml 60°C heißem Wasser gewaschen und schließlich im Vakuum getrocknet.
Ausbeute: 79 g

Das Produkt liefert bei der Einfärbung von Polystyrol in der Masse ein sehr klares Blaugrün.

### Beispiel 2

In einen 1000 ml Sulfierbecher werden 330 ml n-Butanol und 45 ml N-Methyl-2-pyrrolidon eindosiert. Unter Rühren werden 101 g p-Toluidin, 31 g Borsäure und 84 g 99,5%iges Chinizarin und 15 g 93,5%iges Dihydrochinizarin eingetragen. Der Ansatz wird am Wasserabscheider zum Rückfluss erhitzt. Nach 5h am Rückfluss wird der Ansatz abgekühlt, filtriert, mit 160 ml 60°C heißem n-Butanol, danach portionsweise mit 400ml 60°C heißem Methanol, danach mit 4000ml 60°C heißem Wasser gewaschen und schließlich im Vakuum getrocknet.
Ausbeute: 159 g

Das Produkt liefert bei der Einfärbung von Polystyrol in der Masse ein sehr klares Blaugrün.

### Beispiel 3

In einen 1000 ml Sulfierbecher werden 330 ml n-Butanol eindosiert. Unter Rühren werden 45 g Caprolactam, 101 g p-Toluidin, 31 g Borsäure und 84 g 99,5%iges Chinizarin und 15 g 93,5%iges Dihydrochinizarin eingetragen. Der Ansatz wird am Wasserabscheider zum Rückfluss erhitzt. Nach 5h am Rückfluss wird der Ansatz abgekühlt, filtriert, mit 160 ml 60°C heißem n-Butanol, danach portionsweise mit 400ml 60°C heißem Methanol, danach mit 4000ml 60°C heißem Wasser gewaschen und schließlich im Vakuum getrocknet.
Ausbeute: 159 g

Das Produkt liefert bei der Einfärbung von Polystyrol in der Masse ein klares Blaugrün.

### Beispiel 4

In einen 1000 ml Sulfierbecher werden 330 ml n-Butanol und 45 ml N-Methyl-2-pyrrolidon eindosiert. Unter Rühren werden 101 g p-Toluidin, 31 g Borsäure, 1 g p-Toluolsulfonsäure und 84 g 99,5%iges Chinizarin und 15 g 93,5%iges Dihydrochinizarin eingetragen. Der Ansatz wird am Wasserabscheider zum Rückfluss erhitzt. Nach 5h am Rückfluss wird der Ansatz abgekühlt, filtriert, mit 160 ml 60°C heißem n-Butanol, danach portionsweise mit 400ml 60°C heißem Methanol, danach mit 4000ml 60°C heißem Wasser gewaschen und schließlich im Vakuum getrocknet.
Ausbeute: 154 g

Das Produkt liefert bei der Einfärbung von Polystyrol in der Masse ein klares Blaugrün.

### Beispiel 5

In einen 1000 ml Sulfierbecher werden 320 ml n-Butanol eindosiert. Unter Rühren werden 80 g Caprolactam, 101 g p-Toluidin, 31 g Borsäure und 84 g 99,5%iges Chinizarin und 15 g 93,5%iges Dihydrochinizarin eingetragen. Der Ansatz wird am Wasserabscheider zum Rückfluss erhitzt. Nach 5h am Rückfluss wird der Ansatz abgekühlt, filtriert, mit 160 ml 60°C heißem n-Butanol, danach portionsweise mit 400ml 60°C heißem Methanol, danach mit 4000ml 60°C heißem Wasser gewaschen und schließlich im Vakuum getrocknet.
Ausbeute: 153 g

Das Produkt liefert bei der Einfärbung von Polystyrol in der Masse ein sehr klares Blaugrün.

### Beispiel 6

In einen 1000 ml Sulfierbecher werden 240 ml n-Butanol eindosiert. Unter Rühren werden 160 g Caprolactam, 101 g p-Toluidin, 31 g Borsäure und 84 g 99,5%iges Chinizarin und 15 g 93,5%iges Dihydrochinizarin eingetragen. Der Ansatz wird am Wasserabscheider zum Rückfluss erhitzt. Nach 6h am Rückfluss wird der Ansatz abgekühlt, filtriert, mit 160 ml 60°C heißem n-Butanol, danach portionsweise mit 400ml 60°C heißem Methanol, danach mit 4000ml 60°C heißem Wasser gewaschen und schließlich im Vakuum getrocknet.
Ausbeute: 151 g

Das Produkt liefert bei der Einfärbung von Polystyrol in der Masse ein sehr klares Blaugrün.

### Beispiel 7

In einen 1000 ml Sulfierbecher werden 240 ml Toluol eindosiert. Unter Rühren werden 360 g Caprolactam, 101 g p-Toluidin, 20 g Borsäure, 10 g Milchsäure und 84 g 99,5%iges Chinizarin und 15 g 93,5%iges Dihydrochinizarin eingetragen. Der Ansatz wird am Wasserabscheider zum Rückfluss erhitzt. Nach 5h am Rückfluss wird der Ansatz abgekühlt, filtriert, portionsweise mit 400ml 60°C heißem Methanol, danach mit 4000ml 60°C heißem Wasser gewaschen und schließlich im Vakuum getrocknet.
Ausbeute: 127 g

Das Produkt liefert bei der Einfärbung von Polystyrol in der Masse ein Blaugrün.

### Beispiel 8

In einen 1000 ml Sulfierbecher werden 400 g Caprolactam, 101 g p-Toluidin, 20 g Borsäure, 10 g Milchsäure und 84 g 99,5%iges Chinizarin und 15 g 93,5%iges Dihydrochinizarin eingetragen. Der Ansatz wird auf 110°C erhitzt. Nach 5h wird der Ansatz abgekühlt und filtriert, danach portionsweise mit 400ml 60°C heißem Methanol, danach mit 4000ml 60°C heißem Wasser gewaschen und schließlich im Vakuum getrocknet.
Ausbeute: 121 g

Das Produkt liefert bei der Einfärbung von Polystyrol in der Masse ein Blaugrün.

## Patentansprüche

1. Verfahren zur Herstellung von N,N'-disubstituierten 1,4-Diaminoanthrachinonen, **dadurch gekennzeichnet, dass** man 1,4-Dihydroxyanthrachinone mit aliphatischen oder aromatischen Aminen in Gegenwart eines dipolar aprotischen Lösungsmittels, welches eine Löslichkeit in Wasser von wenigstens 30 Gew.-% bei 20°C besitzt, und wenigstens eines zweiten davon verschiedenen Lösungsmittels umsetzt.

2. Verfahren gemäß Anspruch 1, **dadurch gekennzeichnet, dass** es sich bei dem dipolar-aprotischen Lösungsmittel um ein mit Wasser mischbares Lösungsmittel handelt.

3. Verfahren gemäß Anspruch 1, **dadurch gekennzeichnet, dass** es sich bei dem dipolar-aprotischen Lösungsmittel um ein gegebenenfalls substituiertes Formamid, Sulfoxid, Lactam oder Lacton handelt.

4. Verfahren gemäß Anspruch 1, **dadurch gekennzeichnet, dass** es sich bei dem dipolar-aprotischen Lösungsmittel um Dimethylformamid, Dimethylsulfoxid, Butyrolacton, Caprolactam oder N-Methyl-2-pyrrolidon handelt.

5. Verfahren gemäß Anspruch 1, **dadurch gekennzeichnet, dass** es sich bei der dipolar-aprotischen Verbindung um N-Methyl-2-pyrrolidon handelt.

6. Verfahren gemäß Anspruch 1, **dadurch gekennzeichnet, dass** man in Gegenwart von Borsäure arbeitet.

7. Verfahren gemäß Anspruch 6, **dadurch gekennzeichnet, dass** man ohne den Zusatz von Mineralsäuren oder Carbonsäuren arbeitet.

8. Verfahren gemäß Anspruch 1, **dadurch gekennzeichnet, dass** die aliphatischen oder aromatischen Amine primär sind.

9. Verfahren gemäß Anspruch 1, **dadurch gekennzeichnet, dass** die aromatischen Amine der Formel 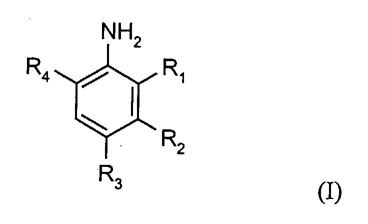 entsprechen, worin
R₁, R₃ und R₄ unabhängig voneinander H oder C₁-C₁₂-Alkyl, insbesondere C ₁-C₄-Alkyl, bedeuten und
R₂ für Wasserstoff oder -SO₂-NH-R₅ steht, wobei R₅ für gegebenenfalls substituiertes Aryl oder Alkyl steht.

10. Verfahren gemäß Anspruch 9, **dadurch gekennzeichnet, dass** R₁, R₂ und R₄ für H und R₃ für Methyl steht.

11. Verfahren gemäß Anspruch 1, **dadurch gekennzeichnet, dass** das zweite Lösungsmittel in Wasser eine Löslichkeit von kleiner 20 Gew.-% bei 20°C besitzt.

12. Verfahren nach Anspruch 1, **dadurch gekennzeichnet, dass** die Löslichkeit des zweiten Lösungsmittels in Wasser zwischen 5 Gew.-% und 15 Gew.-% bei 20°C liegt.

13. Verfahren nach Anspruch 1, **dadurch gekennzeichnet, dass** das zweite Lösungsmittel mit Wasser ein Azeotrop bildet.

14. Verfahren gemäß Anspruch 1, **dadurch gekennzeichnet, dass** das zweite Lösungsmittel mit dem dipolar-aprotischen Lösungsmittel mischbar ist.

15. Verfahren gemäß Anspruch 1, **dadurch gekennzeichnet, dass** das zweite Lösungsmittel ein aliphatischer Alkohol, insbesondere n-Butanol ist.

16. Verfahren gemäß Anspruch 1, **dadurch gekennzeichnet, dass** das dipolar-aprotische Lösungsmittel und das zweite Lösungsmittel im Verhältnis 5 : 95 bis 50 : 50 eingesetzt werden.

17. Verfahren gemäß Anspruch 1, in dem das 1,4-Dihydroxyanthrachinon (Chinizarin) im Gemisch mit seiner Leukoform, dem 2,3-Dihydro-1,4-dihydroxyanthrachinon (Leukochinizarin) eingesetzt wird.

18. Verfahren gemäß Anspruch 17, in dem die Leukoform erst im Reaktionsverlauf erzeugt bzw. zugegeben wird.

19. Verwendung der nach einem Verfahren gemäß wenigstens einem der Ansprüche 1 bis 18 hergestellten Verbindung zum Massefärben von Kunststoffen.
